# EUROPEAN PATENT SPECIFICATION

(11) **EP 1 150 995 B1**
(45) Date of publication and mention of the grant of the patent: **04.01.2006**
(21) Application number: 00904109.6
(22) Date of filing: 12.02.2000
(51) Int. Cl.: C07K 5/04

(54) **A SYNTHETIC PEPTIDE DISTURBING INTRACELLULAR SIGNALING**
SYNTHETISCHES PEPTID ZUR STÖRUNG INTRAZELLULÄRER SIGNALE
PEPTIDE DE SYNTHESE FOURNISSANT UN SIGNAL INTRACELLULAIRE

(30) Priority: 12.02.1999 AU PP864399; 02.06.1999 KR 9920282
(43) Date of publication of application: 07.11.2001
(73) Proprietor: Bukwang Pharmaceutical Co., Ltd., Dongjak-ku Seoul 156-811 (KR)
(72) Inventor: YOON, Jeong, Hyeok, 105-102 Bowon Apartment, Kyunggi-do 449-840 (KR); HAN, Young, Tae, Greenwich, NSW 2065 (AU); LEE, Ky Young, 6-901 Gapohansin Apartment, Seoul 135-272 (KR); KIM, Kilhyoun, Seodaemun-gu, Seoul 120-113 (KR)
(74) Representative: Naumann, Ulrich
(86) International application number: PCT/KR2000/000107
(87) International publication number: WO 2000/047607

(56) References cited:
- US-A- 5 976 819
- L.K. TIMSON GAUEN ET AL.: 'Interactions of p59fyn and ZAP-70 with T-cell receptor activation motifs: Defining the nature of a signalling motif' MOLECULAR AND CELLULAR BIOLOGY vol. 14, no. 6, June 1994, pages 3729 - 3741, XP001037932
- DATABASE WPI Week 199310, Derwent Publications Ltd., London, GB; AN 1993-085859, XP002906280 & US 7 815 749 A (US DEPT HEALTH & HUMAN SERVICE) 01 January 1993
- DATABASE CA [Online] XP002946458 Retrieved from STN Database accession no. 119:7215 & US 7 815 749 A (US DEPT OF HEALTH AND HUMAN SERVICES) 01 January 1993

### SEQUENCE LISTING

<110> YOON, Jeong Hyeok
   HAN, Young Tae
<120> A synthetic peptides disturbing intracellular signaling
<130> PCT000212
<140> 00
   <141> 2000-02-12
<160> 13
<170> Patent In Ver. 2.1
<210> 1
   <211> 11
   <212> PRT
   <213> Artificial Sequence
<220>
   <223> Description of Artificial Sequence: Peptide which specifically binds to SH3 domain with high affinity, thus inhibiting intracellular signal transmission
<400> 1
   Lys Glu Arg Pro Pro Pro Val Pro Asn Pro Asp
    1               5                   10
<210> 2
   <211> 11
   <212> PRT
   <213> Artificial Sequence
<220>
   <223> Description of Artificial Sequence: Peptide which specifically binds to SH3 domain with high affinity, thus inhibiting intracellular signal transmission
<400> 2
   Lys Arg Pro Leu Pro Pro Leu Pro Asn Pro Asp
    1               5                   10
<210> 3
   <211> 11
   <212> PRT
   <213> Artificial Sequence
<220>
   <223> Description of Artificial Sequence: Peptide which specifically binds to SH3 domain with high affinity, thus inhibiting intracellular signal transmission
<400> 3
   Lys Arg Pro Pro Pro Pro Leu Pro Asn Pro Asp
    1               5                   10
<210> 4
   <211> 11
   <212> PRT
   <213> Artificial Sequence
<220>
   <223> Description of Artificial Sequence: Peptide which specifically binds to SH3 domain with high affinity, thus inhibiting intracellular signal transmission
<400> 4
   Lys Glu Arg Pro Pro Pro Leu Pro Asn Pro Asp
    1               5                   10
<210> 5
   <211> 11
   <212> PRT
   <213> Artificial Sequence
<220>
   <223> Description of Artificial Sequence: Peptide which specifically binds to SH3 domain with high affinity, thus inhibiting intracellular signal transmission
<400> 5
   Lys Glu Arg Pro Pro Pro Tyr Pro Asn Pro Asp
    1               5                   10
<210> 6
   <211> 11
   <212> PRT
   <213> Artificial Sequence
<220>
   <223> Description of Artificial Sequence: Peptide which specifically binds to SH3 domain with high affinity, thus inhibiting intracellular signal transmission
<400> 6
   Lys Leu Leu Pro Pro Pro Leu Pro Asn Pro Asp
   1 5 10
<210> 7
   <211> 11
   <212> PRT
   <213> Artificial Sequence
<220>
   <223> Description of Artificial Sequence: Peptide which specifically binds to SH3 domain with high affinity, thus inhibiting intracellular signal transmission
<400> 7
   Lys Arg Pro Leu Pro Pro Leu Pro Leu Pro Asp
   1 5 10
<210> 8
   <211> 11
   <212> PRT
   <213> Artificial Sequence
<220>
   <223> Description of Artificial Sequence: Peptide which specifically binds to SH3 domain with high affinity, thus inhibiting intracellular signal transmission
<400> 8
   Lys Glu Arg Pro Pro Leu Pro Pro Asn Pro Asp
   1 5 10
<210> 9
   <211> 11
   <212> PRT
   <213> Artificial Sequence
<220>
   <223> Description of Artificial Sequence: Peptide which specifically binds to SH3 domain with high affinity, thus inhibiting intracellular signal transmission
<400> 9
   Lys Arg Pro Pro Pro Pro Leu Pro Asn Pro Asp
   1 5 10
<210> 11
   <211> 15
   <212> PRT
   <213> Artificial Sequence
<220>
   <223> Description of Artificial Sequence: Peptide which specifically binds to SH3 domain with high affinity, thus inhibiting intracellular signal transmission
<400> 11
   Lys Glu Arg Pro Pro Pro Val Pro Asn Pro Asp Tyr Glu Glu Leu
   1 5 10 15
<210> 13
   <211> 11
   <212> PRT
   <213> Artificial Sequence
<220>
   <223> Description of Artificial Sequence: Comparative peptide
<400> 13
   Lys Glu Ala Pro Ala Pro Val Ala Asn Pro Asp
   1 5 10

## Description

### BACKGROUND OF THE INVENTION

The present invention relates to synthetic peptides from the motif which binds to src family protein kinase, and pharmaceutical compositions containing the same for treating cancer and immunosuppression.

Many of biological and physiological activation of a cell are controlled by external signals that stimulate or inhibit intracellular events. These events by which an external signal is transmitted into and within a cell to elicit an intracellular response are referred to as signal transduction. Intracellular signal transduction following the extracellular ligation of a wide variety of different types of extracellular factors (For example, hormones, adhesion molecules, cytokines, and the like) involves the activation of protein kinases. In order to activate these molecules, the receptor-target interactions are initially needed. These interactions stimulate a cascade to additional molecular interaction involving multi-pathways that transmit the signal events within the cells.

About 400 different protein kinases are known, and the number of total protein kinases will more than likely double in the few years (Hardie G *et al,* (1995) *The protein kinase Facts Book.* London : Academic). Many of the protein kinases involved in signal transduction consist of multiple domains, some of which have catalytic activity and some of which bind to other cellular proteins. The major area of primary sequence homology for the protein kinases lies within the catalytic domains, referred to as Src homology 1(SH1). The SH1 domains of the protein kinases can be further divided into 11 subdomains shared not only by enzymes but by all protein kinases. The next most common functional domains found are the Src homology 2(SH2) (Sadowski *et al*. (1986) *Mol. Cell. Biol.* 6:4396-4408) and Src homology 3(SH3) domains. SH2 and SH3 domains mediate protein-protein interaction in cellular signaling cascades, and are found in many proteins outside the Src family. Extensive structural and functional studies of SH2 and SH3 domains in signal transduction have recently been described in three dimensional detail, that is pTy-binding (PTB) domains (P Blaikie *et al.,* (1994) *J. Biol. Chem* 269:32 031: Zhou M *et al.,* (1995) *Nature* 92, 7784-7788).

SH2 domains of approximately 100 amino acids are capable of high-affinity binding to phosphotyrosine-containing peptide sequence that promotes protein modification-dependent protein-protein interaction (Pawson *T et al.,* (1992) *Cell.* 70:694-704).

The subsequent discovery that SH2 domains bind to specific phosphorylated tyrosine residues has proincluding Ras GTPase-activating protein(GAP), phosphatidylinositol 3'-kinase(PIK), and phospholipase C-y(Cantley *et al,* (1991) *Cell* 64:281-320). And also, SH2 domains serve to localize these proteins to activated receptors and are implicated in the modulation of enzymatic activity (O'Brien *et al,* (1990) *Mol. Cell. Biol* 10:2855-2862: *Roussel et al*, (1991) *Pro. Natl. Acad. Sci.* USA 88:10696-10700: Bucker *et al*, (1992) *EMBO J.* 11:3469-3479).

Synthetic phosphopeptides based on SH2 domains have been found to block the binding of phosphatidyinositol 3-kinase to the platelet-derived growth factor (PDGF) receptor (Bscobedo *et al*, (1991) *Mol. Cell. Biol.* 11:1125-1132 : Fanll *et al*, (1992) *Nature* 352:726-730). In addition, mutational studies have shown that the SH2 domains of phospatidylinositol 3-kinase, Ras GAP and PLC-γ recognize distinct phosphopeptide sequence in the PDGF receptor (Bant*l et al,* (1992) *Cell* 69:413-423 : Kazlauskas *et al,* (1990) *Science* 247:1578-1581 : (1992) *Mol. Cell. Biol.* 12:2534-2544).

Another common features among the protein kinases are the Src homology 3(SH3) domains. SH3 domains consisting of approximately 60 residues in length and bind to proline-rich peptide sequences have consensus PXXP (wherein, X is a hydrophobic amino acid such as Ile, Leu, Pro, Met, Phe, Tyr) which possess a left-handed polyproline type II helix structure (Feng *et al*, (1994) *Science* 266:1241-1247). The association of SH3 domain with polyproline helix also promotes protein-protein interactions and forms functional oligomeric complexes at defined subcellular sites, frequently in conjunction with other modular domains (Pawson *et al,* (1995) *Nature* 373:573). Proteins can have multiple SH3 domains, potentially allowing clustering of several distinct ligands, and Ser or Thr phosphorylation adjacent to the proline-rich ligand may influence SH3 domain interactions (Chen *et al*, (1996) *J. Biol. Chem* 271:6328). Interestingly, in certain case, SH3-ligand interactions may be negatively regulated by the phosporylation of a conserved tyrosine (Park *H et al*, (1996) *Immunity* 4:515-525: Broome MA *et al*, (1996) *J. Biol. Chem.* 271:16,798-806).

The pTyr-binding (PTB) domains of the She and insulin receptor substrate-1 (IRS-1) proteins recognize phosphopeptide motifs in which pTyr is preceded by residues that form a β turn (usually with the consensus NPXpY) (Blaikie *et al*, (1994) *J. Biol. Chem.* 269:32031). Specificity is conferred by hydrophobic amino acids that lie five to eight residues NH₂-terminal to the pTyr (Trub *et al*, (1995) *J. Biol. Chem.* 270:18205) and therefore recognize their ligands in a distinct manner to form SH2 domain (Zhou *et al*, (1995) *Nature* 378:584). PTB domains may serve a somewhat different role from SH2 domains, because they are found primarily as components of docking proteins that recruit additional signaling proteins to the vicinity of an activated receptor. The PTB domains of proteins such as X11, FE65, and Numb can bind nonphosphorylated peptide motifs, indicating that PTB domains are principally peptide recognition elements, unlike SH2 domains that appear devoted to the job of pTyr recognition.

Protein-protein interactions that are attractive as therapeutic targets include the SH2 domains of Syk family kinases which are essential for coupling with immune response receptors, the SH2 and SH3 domains of Src family kinases which are involved in coupling with receptors and downstream effectors, and the PTB domains which are essential for She functions. The challenges that remain to be met in developing inhibitors of these domains include the need to identity compounds that are highly specific for each protein, that demonstrate membrane permeability and high affinity, and possess the appropriate pharmacokinetics and safety profile.

Protein-protein interactions are involved in each and every step of intracellular signal transduction. Thus, at the plasma membrane, the signal is initiated in the cytoplasm by receptor recruitment of other cellular proteins; in the cytoplasm, the signals are dissiminated to different cellular location; and in the nucleus, the signals are transduced to other proteins involved in transcriptional control from complexes to regulate transcription of particular genes.

The Src-family members share a common regulatory mechanism, but differ in cellular expression and localization. Nine Src-family tyrosine kinases have been identified (Src. Lck, Hck, Fyn, Fgr, Yes, Blk, Lyn, and Yrk) (Brown M (1996) *Biochem Biophys Acta* 1287, 121-149). The highly conserved regulatory apparatus of the Src family member consist of two peptide binding modules : the Src homology domains SH2 and SH3. These modules bind to targets containing phosphotyrosines and polyproline type II helices, respectively, and mediate the formation of protein-protein complexes during signaling. Accumulating evidence suggests that selected sequence surrounding tyrosine phosphorylation site and polyproline rich site can be recognized by proteins containing SH2 domains and SH3 domains, respectively, and that such protein-protein interaction functions to propagate intracellular signal by mediating the function and/or dissolution of protein complexes. Phosphoprotein recognition by SH2 domain and polyproline recognition by SH3 are though to derive specificity from the presence of these domain properties as well as the surrounding amino acid sequence.

US-A-5 976 819 is showing amino acid sequences included within an immunoreceptor tyrosine-based activation motif.

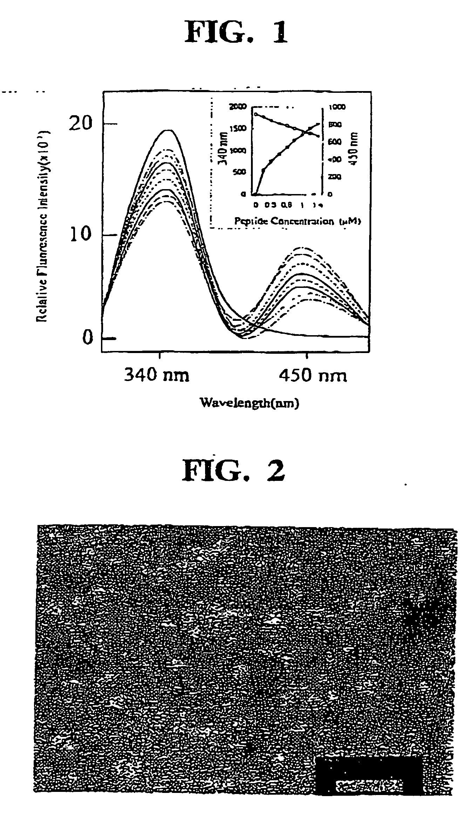

STN International FIZ Karlsruhe, DE. Accession No. 119:7217 & US-A-815 749 and TIMSON GAUEN ET AL. (1994) MOLECULAR AND CELLULAR BIOLOGY, Vol. 14, No. 6, pp. 3729-3741 showing peptide sequences, wherein the peptide sequence may disrupt the intracellular signal transduction mediated by SH2 domain-containing proteins because said peptide inhibits protein-protein interaction. The last mentioned document is further disclosing in figure 1 the CD3 epsilon cytoplasmatic domain which contains an amino acid sequence.

The signaling phenomenon associated biologically or physiologically with cell proliferation can be regulated by inhibiting the protein-protein interaction using peptides which react with small regulatory domains. For example, intra- or extracellular signals which elicit the expression of genes associated with cell proliferation and division is blocked not to be transmitted into nucleus, leading cell growth termination and consequently cell death.

Accordingly, the potential inhibitors of SH3 domains of proteins which are involved in coupling with receptors and transmission to downstream effectors should be met high specificity and high affinity for each protein and possess the appropriate pharmacokinetics and safety profiles.

The present inventors made researches to provide new peptides having high affinity to SH3 domains so as to inhibit protein-protein interactions in signaling pathway, and as a result thereof, they found that synthetic peptides comprising the amino acid sequence (I) show high affinity to SH3 domains, and that, if palmitic acid is coupled to N-terminal of the peptide, the resulting peptides have an increased permeability into cells. The peptide and N-palmitoyl peptide may be advantageously employed for treating cancer and immune suppression-associated diseases.

### SUMMARY OF THE INVENTION

Thus, the present invention provides novel inhibitor peptides according to claim 1 showing high affinity to SH3 domains and capable of disturbing or blocking intracellular signal pathway mediated by SH3-containing proteins, while possessing appropriate pharmacokinetics and safety profiles.

The present invention still provides pharmaceutical composition according to claim 2 for treating cancer or immune suppression-associated diseases, which comprises the peptide of the present invention.

### BRIEF DESCRIPTION OF THE DRAWINGS

FIG. 1 is a graph showing binding affinity the inhibitor peptides of the invention to SH3-GST fusion protein;
FIG. 2 is a fluorescent microscopy of translocation of palmitoylated inhibitor peptides the cells; .
FIG. 3 is confocal microscopy showing distribution of palmitoylated inhibitor peptides within cells;
FIG. 4 is an electrophoresis showing the effect of palmitoylated inhibitor peptides on the phosphorylation of ryrosine;
FIG. 5 is an electrophoresis showing the effect of palmitoylated inhibitor peptides on the phosphorylation of components of signaling pathway;
FIG. 6 is flow cytometry showing the effect of inhibitor peptides on the intracellular calcium concentration;
FIG. 7 is a graph showing the effect of inhibitor peptides on cancer cell growth, which is measured by MTS analysis; and
FIG. 8 is microscopic photos showing the changes in the number and shape of cancer cells treated with inhibitor peptides of the invention.

### DETAILED DESCRIPTION OF THE INVENTION

The synthetic peptides of the present invention which are derived from the motifs binding to certain proteins, for example Src family kinases (hereinafter, referred to as "peptide" or "inhibitor peptide"), have part or all of the amino acid sequence represented by the following formula (I) :

KX₁X₂X₃PX₄X₅PX₆PX₇ (I)

Wherein, K is Lys;
P is Pro,
X₁, is Glu, Arg, Asp or Lys;
X₂ is Arg, Lys, Pro, Leu or Ile;
X₃, X₄ and X₅ are, identical or different from each other, independently Pro, Met, Val, Trp, Phe, Ile or Leu;
X₆ is Asn, Gln, Arg or Lys;
X₇ is Asp, Glu, Arg or Lys; and
wherein one of these amino acid residues may be conjugated to 1 or 4 amino acid residues, wherein in the case that one residue is conjugated, the amino acid is Tyr, pTyr(Phosphotyrosine), and that four residues are conjugated, the amino acids may be Tyr or pTyr for the first residue, Asp or Glu for the second and third residues, and hydrophobic amino acids such as Ile, Leu, Val or Met for the fourth residue.

The representative peptides having all or part of the sequence (I) of the present invention may include SEQ. ID. NOs 1 through 12, which are indicated in Sequence Listing.

The peptides of the present invention, which bind target protein within cells, are determined by *in vitro* binding assay using GST-fusion protein expressing SH3, and deteriorate certain cells, e.g. cancer cells expressing Src family kinases.

The peptides according to the present invention may be manufactured chemically or by gene-recombination technology, and their variants and derivatives produced by modification such as deletion, substitution and addition. They preferably have 4 to 20 amino acid residues.

The pharmaceutical composition containing the inhibitor peptides may be employed in the form of solutions, micelles, liposome, or suspensions in an appropriate adjuvant. The inhibitor peptide may be conjugated to lipid in order to enhance its translocation into cells and targeting to cancer cells. In a preferred embodiment, the peptide may be conjugated with mono- or di-palmitic acids to its N-terminal lysine.

The pharmaceutical composition according to the present invention may be administered to human via parenteral or local routes, and preferably in the form of solution or suspension in aqueous vehicles.

The inhibitor peptides may be employed for antitumor treatment for hepatocellular carcinoma and other cancers which express SH3 domains, as well as to suppress the metastasis of cancer cells after clinical surgery in cancer patients.

### Definition of Terms

Throughout the specification and claims, the terms have the following meanings:

The term "peptide" is used interchangeably with "oligopeptide" in the present specification to designate a series of residues, connected one to the other typically by peptide bonds between the alpha-amino and carbonyl groups of adjacent amino acids. The length of oligopeptides is not critical to the invention so long as the correct sequences are maintained. The peptides are typically less then about 50 residues in length, except for repeatedly conjugated forms (example for SH2-SH2-SH2----n, SH3-SH3-SH3-----n), and usually consist of between about 4 and about 20 residues, preferably 13 or 16 residues.

"Inhibitor peptides" are peptides which comprise SH2 and SH3 binding domains such that the peptide will bind the intracellular proteins contained these domains and be capable of blocking or disturbing signal pathway events. Thus, in a variant of cancer cell, inhibitor peptides are capable of binding to an appropriate intracellular protein such as protein kinase and signal adoptive protein which are related with signal pathway for the cell proliferation and division.

The term "palmitoylated peptide" refers to the pattern of lipid (palmitic acid)-conjugated inhibitor peptide. The palmitoylation is designated to enhance the translocation of inhibitor peptides into the cells and the targeting cancer in vivo.

The "cancer" includes a variant of human/animal cancers that express intracellular proteins containing SH2, SH3 and PTB domains.

Moreover, three letter codes are used to indicate amino acid residues according to WIPO Standard ST.25(1998), Appendix 2, Table 3.

### Free Texts (<223>) in Sequence Listing

SEQ. ID. NO.1 ~ SEQ. ID. NO. 12 : Peptide which specifically binds to SH3 domain with high affinity, thus inhibiting intracellular signal transmission.

### SEQ. ID. NO. 13 : Comparative peptide

The present invention will be described in more detail below.

The present invention provides peptides capable of inhibiting signal transduction pathway in cancer. The subdomains of inhibitor peptides are originated from the multi-domains of proteins binding to particular proteins such as Src family kinases, and show high affinity to SH3 domains so that can be used to regress/reject cancer cells by inhibiting intracellular signal pathway mediated by SH3 domains.

The peptides of the invention can be prepared synthetically or by recombinant DNA technology. Although the domains comprising inventive peptides will preferably be substantially free of other naturally occurring host cell and cancer cell, such as lymphocytes and a variety of cancer cells, other host cells, proteins and fragments thereof, in some embodiments the peptides can be synthetically conjugated to native fragments of particles. The peptides may be prepared by recombinant DNA technology in which expression vectors into which DNA having DNA sequence coding for the peptide is incorporated are employed. Such vectors can be constructed so as to be transferred to cancer-inducing loci, transfected into an appropriate host and then expressed under proper conditions according to the methods described by Sambrook *et al*. (Molecular Cloning, 1989, Cold Spring Harbor, Cold Spring Harbor Laboratory Press). Moreover, the peptides may be prepared from fusion proteins containing one or more peptide fragments of the invention.

As the coding sequence for peptides of the length contemplated herein can be synthesized by chemical techniques, for example, the phosphotriester method of Matteueei et al., J. Am. Chem. Soc. 103:3185 (1981), modification can be made simply by substituting the appropriate base for those encoding the native peptide sequence. The coding sequence can then be provided with appropriate linkers and ligated into expression vectors commonly available in the art, and the vectors used to suitable host to produce the desired fusion protein. A number of such vectors and suitable host system are now available. For expression of the fusion proteins or polypeptides, the coding sequence will be provided with operably linked start and stop codons, promoter and terminator regions and usually a replication system to provide an expression vector for expression in the desired cellular host.

The polypeptides or peptides can be of a variety of lengths, either in their neutral (uncharged) forms or in forms which are salts. They may be free of any modifications, or may be subject to modifications such as glycosylation, side chain oxidation, or phosphorylation as long as their biological activity retains. The peptides have a length of about 50 amino acid residues or less, preferably 4 to 20 amino acid residues, and most preferably 8 to 16 amino acid residues.

The *in vivo* activity of the inventive peptides comprising the domains involved in intracellular signaling cascades can be increased by modification such as lipidation, glycosylation or conjugation to other peptides. For instance, a covalent attachment of lipid chains such as palmitic acid to peptides will increase intracellular translocation and cancer targeting by the affinity with lipid. In addition, the use of cancer or specific organ targeting vector/viral vector, which are encoding to these inventor forms, can also be applied the same purpose. The lipids, which may be conjugated to the peptide of the invention in order to increase the translocation of the peptide into cells by enhancing the affinity with cell membrane lipids, may include C₁₈ saturated fatty acids, or C₁₆ or C₁₈ unsaturated fatty acids.

The peptides can also be modified by extending or decreasing its amino acid residues, for example by the addition or deletion of amino acids. The peptides of the invention can also be modified by altering the order or composition of certain residues as long as such modification does not affect the activity of the peptide. Those of ordinary skill in the art to which the present invention pertain readily appreciate that the amino acid residues ("critical amino acids")essential for biological inhibitory activity, for example those at critical contact sites or conserved residues, are not subject to such alteration to retain their inhibitory activity. The non-critical amino acids need not be limited to those naturally occurring in proteins, such as L-a-amino acids as well, such as β, γ, δ amino acids, as well as many derivatives of L-a-amino acids.

Typically, a series of peptides with single amino acid substitutions are employed to determine the effect of electrostatic charge, hydrophobicity, etc, on binding. For instance, a series of positively charged (e.g. Lys or Arg) or negatively charged (e.g. Glu) amino acid substitutions are made along the length of the peptide revealing different patterns of sensitivity towards intracellular kinases/adopter proteins containing signaling modules SH2 or SH3. The number and types of residues which are substituted or added depend on the spacing necessary between essentially contact points and certain functional attributes which are sough (e.g. hydrophobicity versus hydrophilicity). Increased binding affinity for a appropriated intracellular target proteins involved in signal pathway may also be achieved by such substitutions, compared to the affinity of the intact peptides of the invention. In any event, such substitutions should employ amino acid residues or other molecular fragments chosen to avoid, for example, steric and charge interference which might disrupt binding.

Amino acid substitution is typically of single residue. Substitutions, deletions, insertions or any combination thereof may combined to activate a final peptide. Substitutional variants are those in which at least one residue of a peptide has been removed and a different residue inserted in its place. Such substitutions generally are made in accordance with the following Table 1 when it is desired to finely modulate the characteristics of the peptide.

**TABLE 1**

| Suitable residues for amino acid substitutions | |
|---|---|
| Original residues | Exemplary Substitutions |
| Ala | Ser |
| Arg | Lys |
| Asn | Gln: His |
| Asp | Glu |
| Cys | Ser |
| Gln | Asn |
| Glu | Asp |
| Gly | Pro |
| His | Asn: Glu |
| Ile | Leu: Val |
| Leu | Ile: Val |
| Lys | Arg: Gln: Glu |
| Met | Leu: Ile |
| Phe | Met: Leu: Tyr |
| Ser | Thr |
| Thr | Ser |
| Trp | Tyr |
| Tyr | Trp: Phe |
| Val | Ile: Leu |

Substitutional alterations cause a significant change in the functions of the proteins, for example the affinity toward kinases or adoptor proteins. Substitution may produce a big impact on the structure of the peptide backbone, for example as a sheet or helical conformation, the charge or hydrophobicity of the molecule at the target site or the bulkiness of the side chain. The substitutions which in general are expected to produce the greatest changes in peptide properties will be those in which a hydrophilic residue, e.g. seryl, is substituted for a hydrophobic residue, e.g. leucyl, isoleucyl, phenylalanyl, valyl or alanyl; a residue having an electropositive side chain, e.g. lysyl, arginyl, or histidyl is substituted for an electronegative residue, e.g. glutamyl or aspartyl; or a residue having a bulky side chain is substituted for one not having a side chain, e.g. glycine

All the peptides in which one or several amino acids are substituted with D-amino acids, or with amino acid derivatives such as hydroxyproline, hydroxylysine, cystine, thyroxine, norleucine or pyroglutamic acid, or with methylated amino acids, are encompassed within the scope of the present invention. Moreover, the peptides which have C-terminal of an amide, a carboxyl or an ester also fall in the scope of the present invention as long as they can accomplish the object of the invention.

The peptides of the present invention may also comprise isosteres of two or more residues. An isostere as defined here is a sequence of two or more residues that can be substituted for a second sequence due to the steric conformation of the first sequence. The term specifically includes peptide backbone modifications well known to those skilled in the art. Such modifications include modifications of the amide nitrogen, the α-carbon, amide carbonyl, complete replacement of the amide bond, extensions, deletions or backbone crosslinks.

Modification of peptides with various amino acid mimetics of unnatural amino acids are particularly useful in increasing the *in vivo* stability of the peptide. Stability can be assayed in a number of ways. For instance, peptidase and various biological media, such as human plasma and serum, have been used to determine stability.

The present invention also provides pharmaceutical compositions containing one or more peptides of the invention as active ingredients.

In some embodiments it may be desirable to include in the pharmaceutical compositions of the invention at least one component which assists the intracellular translocation and cancer targeting using peptide-lipidation method. Lipids have been identified as agents capable of assisting the intracellular translocation and partially cancer targeting. For example, palmitic acid residues can be attached to the alpha and epsilon amino groups of Lys residues and then linked, e.g. via one or more linking residues such as Gly, Gly-Gly, Ser or Ser-Ser. The lipidated peptide can then be injected directly in the form of micelle, or incorporated into a liposome or emulsified in an adjuvant, e.g. incomplete Freund's adjuvant. In a preferred embodiment, a particularly effective translocation and targeting comprises palmitic acid attached to alpha or epsilon amino groups of Lys. Inhibitor peptides of the invention can be coupled, for example to Pal₂K (Palmityl-Palmityl-Lys or Pal₃CK(Palmityl-Palmityl-Palmityl-Cys-Lys), and the resulting lipopeptides can be administrated to the subject to specifically inhibit cancer growth.

For targeting to the cancer cells, the peptides may be incorporated into the liposome which contains antibody or its fragments specific for cell surface determinants on the target cancer cells.

For solid formulations, pharmaceutically acceptable conventional nontoxic solid carriers may be used. Such carriers may include, but not limited to, mannitol, lactose, starch, magnesium stearate, sodium saccharin, talcum, cellulose, glucose, sucrose, magnesium carbonate, and the like. For oral administration, 10-95% by weight, and more preferably 25%-75% by weight based on the total weight of the formulation of one or more peptides of the invention as active ingredients are incorporated into the above listed carriers to give oral formulations.

The pharmaceutical composition of the present invention may also be administered into the subject by parenteral or local route, for example intravenous, subcutaneous, intracutaneous, intramuscular route and the like.

For aerosol administration, the peptides of the present invention are preferably supplied in finely divided form along with a surfactant and propellant. Typical amount of peptides is 0.01%-20% by weight, and preferably 1%-10% by weight based on the total weight of the formulation.

The amount of the composition of the present invention to be administered is not limited to certain range, and may be determined by those of skilled in the an without difficulty depending on the severity, condition, age and body weight of the patient. But, it is generally in the range of about 5 - 100 mg/kg/day, particularly about 10 - 50 mg/kg/day, and preferably about 30mg/kg/day.

The peptides or pharmaceutical composition containing the same may show physiological activity in living body and its physiological activity may be measured *in vivo* in blood or bone marrow samples from patients administered with the peptides or compositions.

The present invention will be described in more detail by way of representative working examples, but should not be construed to limit in any way the scope of the present invention.

### EXAMPLES

### Example 1 : Peptide synthesis.

Inhibitor peptides (SEQ ID NO: 1) was synthesized by solid-phase peptide synthesis using the Applied Biosystems 430 A synthesizer, our own Fmoc program and Fmoc L-amino acids from Novabiochem (Laufelfingen, Switzerland) with side-chain-protecting groups: Lys (Boc), Arg (Mtr), Asp (OtBu), Tyr (tBu).

Starting each synthesis with 1 g p-benzyloxybenzylalcohol-resin loaded with either Fmoc-Asp-OH (0.35 mmol) or Fmoc-Arg-OH (0.35 mmol) the following synthetic cycles were carried out: N^{α}-deprotection with 55% piperidine in N-methylpyrrolidone (1x2 min, 1x5 min); preactivation of Fmoc-AA-OH (1.5 mmol) in N-methylpyrrolidone (6 ml) with diisopropylcarbodiimide (1.5 mmol) and 1-hydroxybenxotriazole (1.5 mmol) followed by coupling for 1.5 hours. After washing with N-ethylmorpholine (5% in N-methylpyrrolidone), preactivation and coupling were repeated. Capping of non-reacted amino groups was performed with acetic anhydride (2.5 mmol) and diisopropylethyamine (1.2 mmol) in N-methylpyrrolidone. Having synthesized the resin-bound peptides, analytical peptide probes were removed by trifluoroacetolytic cleavage and checked by high performance liquid chromatography, FAB mass spectrometry amino acid analysis

The same procedure was repeated to synthesis the peptides of SEQ. ID. NO. 2 through SEQ. ID. NO. 12.

### Example 2 : Lipidation

Peptide-conjugated resin (SEQ. ID. NO. 1) and palmitic acid were coupled in benzotriazole-1-yl-oxy-tris-pyrrolidino-phosphonium hexafluorophosphate / N-hydroxybenzotriazole / N-methylmorpholine (2:1:2), and added in a fivefold excess to the peptide resins. After 3 hours, the lipopeptide-resin was washed with methanol (3 times) and couplings were shown to be complete to 99% by the ninhydrin test. The less coupled peptide was double-coupled using a fivefold excess in benzotriazole-1-yl-oxy-tris-pyrrolidino-phosphonium hexafluorophosphate / N-hydroxybenzotriazole / N-methylmorpholine (2:1:2) for about 14 hours. The lipopeptides were removed from the resin in trifluoroacetic acid (4 ml) containing thioanisol (250 µl) and ethanedithiol (150 µl) within 4 h. The filtrate was evaporated the residue taken up in acetic acid and poured into stirred cold ether. The precipitated lipopeptide was washed three times with ether. Further purification was achieved by precipitation from trifluoroethanol/chloroform (1:3) (3 ml) with cold acetone (20 ml) followed by addition of a few drops of water. The lipopeptides were lyophilized from t-butanol/water (3:1). Amino acid analysis and FAB-mass spectrometry proved the identity of the lipopeptides each having palmitoyl-palmitoyl at the N-terminal lysine of the peptides of SEQ. ID. NO. 1 through SEQ. ID. NO. 12.

### Reference Example : Preparation of GST fusion proteins

In order to assess the inhibitory activity of the present peptides, the ability of the inhibitor peptides to inhibit the interaction between a specific peptide motif and SH3 domain was determined. Various SH2 and SH3 domains were subcloned in frame with the pGEX vector system (Pharmarcia). The resulting pGEX-E2 construct encoded a glutathione-S-transferase (GST)/SH2 and glutathione-S-transferase (GST)/SH3 fusion protein. The pGEX construct was introduced into E.coli by transformation, and the transformation grown in liquid media (LB) in the presence of IPTG. Purification of GST/E2 fusion protein was by standard protocols (Current Protocols in Molecular Biology, eds. Ausubel et. al., (NY:John Wiley and Sons, 1991). Briefly, the cells were pelleted and resuspended in buffer A (50 mM TRIS (pH7.5), 2 mM EDTA, 250 mM NaCl, 5% glycerol (V/V), 1% Tween (V/V), 1% Triton X-100 (V/V), to which 2 mM PMSF and 15 mM 2-mercaptoethanol were added. Bacteria were mechanically disrupted by sonication or by microfluidizer, and the soluble fraction of the lysate was absorbed with glutathione-agrose beads which had been rehydrated in buffer A. Absorption was for 30 min to 14 hr at 4°C during which the beads were maintained in suspension by rocking. Subsequently, the beads were washed 3 times in cold buffer A followed by 5 washes in 50 mM Tris, pH 8.0.

### Experimental Example 1 : Measurement of binding of Peptides to SH3 domains

To assess the ability of the peptides prepared in Examples 1 and 2 to bind to SH3 domains, the peptide ("P1", SEQ. ID. NO. 1) and its palmitoylated product ("LP1") were coupled to aminomethyl coumarine acetate ("AMCA").

Each peptide 5 mg and AMCA were dissolved into 1ml of 50 mM sodium bicarbonate buffer (pH 8.5), and 5 mg/ml dimethylsulfoxide (DMSO) was added thereto just before starting reaction. 50 - 100 µl of AMCA was gradually added to the reaction mixture while stirring or agitation, and the resulting mixture was allowed to react at 4°C for a day with continuous stirring. After reaction, peptides coupled to AMCA were separated from non-reacted AMCA by gel filtration.

Then, the AMCA-peptide complex (0.075-1.4µM) was reacted with 3.12 µM GST/SH3 fusion protein, and the reaction mixture was allowed to stand at room temperature for 1 minute. The binding affinity between the peptide of the AMCA-peptide complex and SH3 domain of GST/SH3 fusion protein by using resonance energy transfer method and double laser spectrofluorometry (PTL-3965, Japan). This method is based on the phenomenon that the peptide bound to AMCA emits fluorescence at 350-450 nm. The relative transfer efficiencies were determined as follows : the sample containing the GST/SH3 fusion protein and peptide-AMCA complex was irradiated at 280 nm to excite tryptophan of GST-SH3 fusion protein with irradiation at 280 nm, and AMCA-peptide complex was excited by the emission of 350nm by the excited tryptophan to emit 450 nm fluorescence.

As result, when the peptide is bound to AMCA and then to SH3 domain of GST fusion protein, the 350 nm fluorescence of tryptophan by 280 nm emission reduces while the 450 nm fluorescence of AMCA coupled to the peptide increases (FIG. 1). It can be explained that the peptide of the complex binds to GST/SH3 fusion protein to absorb the fluorescence emission wavelength of 350 nm produced by tryptophan of GST/SH3 fusion protein, thus making it possible for peptide of the complex to emit 450 nm fluorescence.

The 450 nm fluorescence emission of AMCA increases proportionally and the 350 nm fluorescence emission of tryptophan decreases depending on the amount of the peptide of the complex, while 450 nm fluorescence of GST/SH3 fusion protein does not show such dependency. It consequently means that the peptides of Examples 1 and 2 have high affinity to SH3 domain.

### Experimental Example 2: Detection by fluorescent microscopy of translocation of palmitoylated inhibitor peptides within cells

In order to determine the translocation of the above palmitoylated inhibitor peptides within cells, cancer cells were treated with these peptides.

As a result seen in Figure 2 fluorescence can be observed in specific regions in the cancer cells.

### Experimental Example 3: Confocal microscopy showing distribution of palmitoylated inhibitor peptides within cells

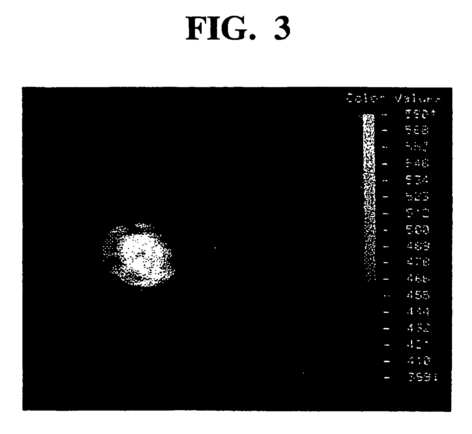

The precise distribution of palmitoylated inhibitor peptides in cells is shown in Figure 3.

### Experimental Example 4 : Measurement of the inhibition effect on phosphorylation of intracellular proteins

In order to test whether palmitoylated peptides can modulate intracellular signal pathway, their effects on signal pathway mediated by T cell receptor ("TCR") was measured. The TCR-mediated signal pathway is activated by phosphorylation of CD3 and ξ ITAM(Immunoreceptor Tyrosine based Activation Motifs)s by Src kinase such as Lck and then the phosphorylated CD3 and ξ ITAMs react with ZAP-70 to phosphorylate tyrosine of ZAP-70.

A mixture of Jurkat cell and inventive peptide (palmitoylated peptide SEQ- ID. NO. 1; "LP1"), palmitoylated ("I.Pc") or non-palmitoylated ("Pc") comparative peptide (SEQ-ID. NO. 13 was incubated at 37°C for 12 hours. The cell lysate was subjected to blotting using antibody against phophotyrosine.

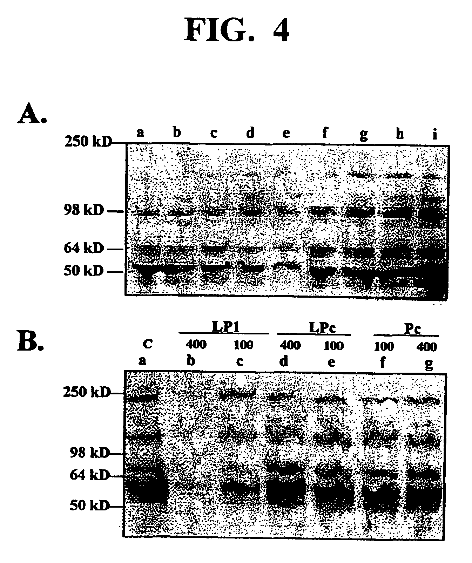

As result shown in FIG. 4, for the LP1 treated group, phosphorylation of tyrosine within cells drastically decreases at 100 µg/ml or 400 µg/ml of LP1 (lane b and lane c in FIG. 4B), while the LPc- or Pc-treated groups show basic level of phosphorylation. FIG. 4A shows the result when the concentration of LP1 is varied between 2 µg/ml and 200 µg/ml for the incubation of 6 hours. The tyrosine phosphorylation decreases at the concentration of 50-100 µg/ml (lane c and lane d in FIG. 4A), and drastically decreases at the concentration of about 200 µg/ml (lane e.Z in FIG. 4A).

These results prove that the peptides of the present invention can effectively inhibit the early stage of signal pathway initiated by tyrosine phosphorylation. In addition, even though not depicted in Figure, the palmitoylated peptide (LP1) shows higher inhibitory effect on the tyrosine phosphorylation than non-palmitoylated peptide, suggesting that the palmitoylation can enhance the translocation efficiency of peptide into cells.

### Experimental Example 5 : Analysis of effect of peptide on phosphorylation of components of signal pathway

As can be seen from the results of Experimental Example 2, it is believed that the palmitoylated peptide of the present invention inhibits the components involved in the early stage of signal pathway induced by cytoplasmic phosphotyrosine. Among the various early stage phenomena associated with signal pathway of T cells such as Jurkat cell, tyrosine phosphorylation is modulated by Src family kninase such as Lck or Fyn, while dephospohorylation is modulated by protein tyrosine phosphatase. Then, crosslinking of TCR in T cell and CD3 phosphorylation by Src kinase occur, and the phosphorylated CD3 complements ZAP-70. ZAP-70 binds to phosphorylated ITAMs in CD3 chain and Zeta chain to accelerate the accumulation of phsophorylated Zeta chain as well as to block dephosphorylation by tyrosine phosphatase. Accordingly, the inhibition of Zeta chain phosphorylation by LP1 is accomplished by directly blocking Lck function or indirectly blocking the binding of ZAP-70 to phosphorylated ITAMs. Thus, the effect of LP1 or Pc can be determined by measuring the degree of phosphorylation of Lck, ZAP-70, CD3 or Zeta.

Jurkat cell was treated with 50 µg/ml or 300 µg/ml of palmiloylated peptide for 6 hours and then suspended in a buffer containing leupeptin, aprotinin, phospharase inhibitor (2 mM sodium orthovanadate, 0.4 mM EDTA, 10 mM sodium fluride and 10 mM sodium pyrophsphate), 50 mM TRIS-hydrochloride (pH 8.0), 300 mM sodium chloride and 0.5% Triton X-100. Cell lysate was subjected to electrophoresis on SDS-polyacryl amide gel (4-20% SDS-PAGE) and then transferred to nitrocellulose membrane.

Nitrocellulose membrane was treated with 4% skim milk in PBS buffer and subjected to immunoblotting using the antibody indicated below to evaluate the expression at protein level. As antibody, clone 4G10 anti-phophotyrosine antibody (Upstate), anti-ZAP-70 antibody (Transduction Laboratories), anti-Lck antibody (Santa Cruz) or anti-Zeta(ξ) chanin antibody (Santa Cruz), which was diluted to 1:1000, 1:1000, 1:1000 or 1:500 respectively, and secondary antibody coupled to HRPO (Amersham) in 1:2000 dilution were employed.

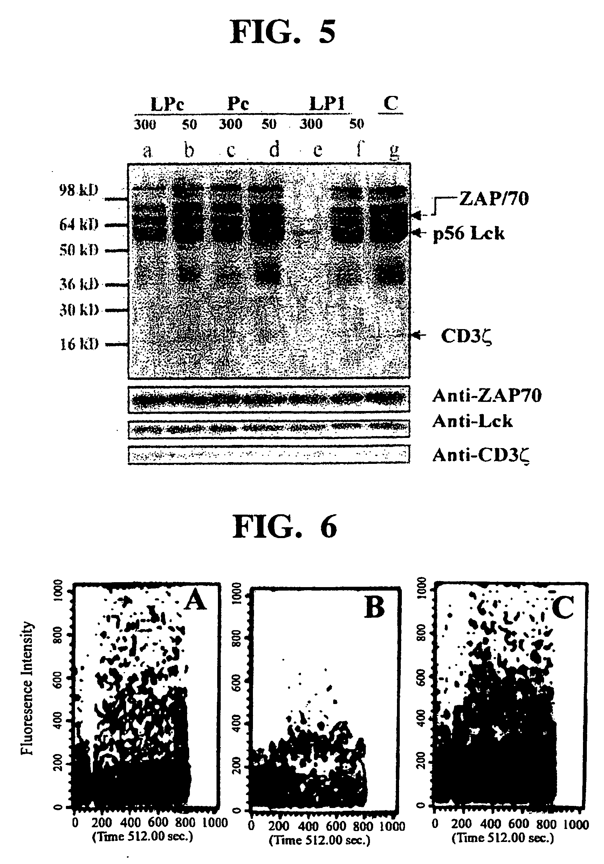

As result, Lck kinase activity was significantly reduced by LP1, but not by LPc or Pc (FIG. 5). In FIG. 5, the terms of "LP1", "LPc" and "Pc" have the same meaning as defined above in FIG. 4, and "C" indicates for control group. The phosphorylation of CD3 Zeta also was significantly reduced by LP1, indicating that the phosphorylation of CD3 Zeta was influenced negatively by the decrease in the Lck kinase activity.

The inhibitory effect of LP1 is even stronger on ZAP-70. The inhibition of ZAP-70 phosphorylation causes a change in dynamics of phosphorylation, and in particular, the inhibitory action on Lck-induced ZAP-70 phosphorylation was strongest when the concentration of LP1 was 200 µg/ml or more. Thus, it can be found that LP1 blocks the early stage signal pathway in Jurkat cells by inhibiting Src kinase, and it shows strongest inhibition when applied to a concentration of about 200 µg/ml or more per 1 x 10⁶ cells.

### Experimental Example 6 : Analysis of effect on intracellular Ca⁺⁺ transport

As can be seen from Experimental Example 3, peptides of the present invention inhibit phosphorylation of Lck and other related proteins, which eventually influences the late stage phenomena of signal pathway such as calcium ion transport within cell.

In order to determine whether the peptides of the present invention inhibit the late stage of signal pathway, Jurkat cells were treated with LP1 (100 µg/ml) at 37°C for 1 hour (FIG. 6B), other peptides (FIG. 6C) or buffer only (FIG. 6A). The treated cells were resuspended in PRMI1640 medium supplemented with 3 mM Fluo-3AM to a concentration of 5 × 10⁶ cells/ml and then incubated at 37°C for 30 minutes. The cells were washed with ice-cold LOCKS buffer (150 mM sodium chloride, 1 mM magnesium sulfate, 5 mM potassium chloride, 10 mM glycine, 15 mM HEPES, pH 7.4) and then resuspended in the same buffer to a final concentration of 1 × 10⁶ cells/ml. Thus-treated cells were preserved on ice in the dark before use. For flow cytometric analysis, cells were heated to 37°C for 5 minutes, and the calcium level before and after addition of anti-CD3 antibody (OKT3) was measured by FACScalibur (Becton-Dickinson, San Jose, CA) (FIG. 6).

As result, for the group treated with LP1, the outflow of calcium ion which follows the stimulation of TCR by OKT3 does not occur, while LP1 treatment does not influence the calcium ion inflow induced by ionomycin. This facts indicate that the peptide of the present invention influences the downstream of signal pathway such as intracellular calcium ion level in TCR-mediated signal pathway.

### Experimental Example 7 : Anticancer activity : Measurement of cell growth with MTS assay.

To test anticancer effect in vitro, human hepatocellular carcinoma (SNU-398)(Park J G *et al* (1995) *Int. J. Cancer* 62:276-282) was obtained from Korea Cell Line Bank (Korea). Hepatocelluar carcinoma (HCC) was maintained in culture using RPMI-1640 (Sigma) medium (Gibco BRL) and 5 x 10⁻⁵ M 2-mercaptoethanol (Sigma).

SNU-398 cells (5 x 10⁵ cell/well) were plated in a 96 well plate. Assay medium consisted of RPMI-1640 (Sigma) contained 10% FBS (Gibco BRL), 4 mM L-glutamine (Sigma), gentamicin (Gibo BRL) and 5 x 10⁻⁵ M 2-mercaptoethanol (Sigma). Palmitoylated inhibitor peptides (LP1 and LP2) were treated with varying concentration and then incubated for 24 hours in CO₂ incubator at 37°C. Then 20 µl/well of CellTiter 96® AQᵤₑₒᵤₛ solution reagent (Promega) were added. After 1-2 hour at 37°C incubator in a 5% CO₂, the absorbance at 490nm was recorded using an ELISA plate reader(Bio-Rad). Each point represents the mean± SD of 3 replicates. The background absorbance shown at zero cells/well was subtracted from these data.

For comparison, the same experiments were carried out by using the comparative peptide ("Pc"; SEQ. ID. NO. 13) and palmitoylated Pc("LPc").

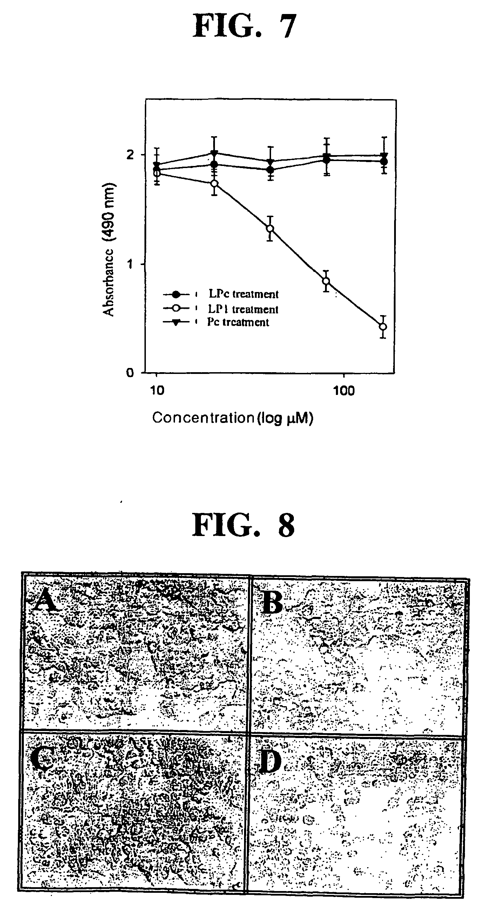

The growth of cancer cell treated with LP1 was significantly decreased, and in particular began to show decrease at 30 µg dose and reach the maximum decrease at 160 µg dose where the cell was reduced to 1/4 of its original number (FIG. 7). FIG. 8 is the microscopy of the cell growth change with peptide treatments, which shows that the LP1 treatment (8D) induces a significant decrease in the density of cancer cells and morphological change of cancer cells, while control (8A), Pc treatment (8B) and LPc treatment (8C) show no or only insignificant changes. The cancer cells treated with LP1 reduced in their size and lost adherence to the plate.

The above results suggest that the inhibitor peptides of the present invention show potential inhibitory effect on the proteins having SH3 domains, which are involved in signal transduction associated with mitosis as well as signaling associated with Lck and the like. Accordingly, the peptides of the invention can be effectively employed as anticancer agents.

### OTHER REFERENCES

Volpina .O. M., et al., Vaccine 14:1375-1380 (Oct., 1996), "Synthetic vaccine against foot-and-mouth disease based on a palmitoyl derivative of the VP1 protein"

Frankenburg. S., et al., Vaccine 14:923-929 (Jun., 1996), "Effective immunication of mice against cutaneous leishmaniasis using an intrinsically adjuvanted synthetic lipopeptide vaccine"

Deprez. B., et al., Vaccine 14:375-382 (Apr., 1996), "Comparative efficiency of simple lipopeptide constructs for in vivo induction of virus-specific CTL"

Babu. J. S., et al., Vaccine 13:1669-1676 (Dec, 1995), "Priming for virus-specific CD8+ but not CD4+ cytotoxic T lymphocytes with synthetic lipopeptide is influenced by acylation units and liposome encapsulation"

Sauzet. J. P., et al., Vaccine 13:1339-1345 (Oct, 1995), "Long-lasting anti-viral cytotoxic T lymphocytes induced in vivo with chimeric-multirestricted lipopeptides"

Rouaix. F., et al., Vaccine 12:1209-1214 (Oct, 1994), "Effect of a lipopeptidic formulation on macrophage activation and peptide presentation to T cells"

Celis. E., et al., P.N.A.S. (USA) 91:2105-2109 (Mar., 1994), "Induction of anti-tumor cytotoxic T lymphocytes in normal humans using primary culture and synthetic peptide epitopes"

Pawson. T., et al., Cell. 70:694-704 (1992), "SH2 and SH3 domains: from structure to function"

Feng. S., et al., Science 266:1241-47 (1994), "Two binding orientations for peptides to the Src SH3 domain: development of a general model for SH3 ligand interactions"

Burke. T. R ., et al., Drugs of the Future 17:119-31 (1992), "Protein-tyrosine kinase inhibitors"

Tsuruga. T., et al., Chem. Pharm. Bull. (Tokyo) 39:3276-8 (1991) " Biologically active constitiuents fo Melaleuca leucadendron: inhibitors of induced histamine release from rat mast cells"

Hanke. J. K., et al., J. Biol. Chem. 271:697-701 (1996) "Discovery of a novel, potent, and Src family selective tyrosine kinase inhibitor"

Zhu.Q., et al., J. Exp. Med. 180:461-70 (1994), "Deletion within the Src homology domain 3 of Bruton's tyrosine kinase resulting in X-linked agammaglobulinemia (XLA)"

Rickles. R. J., et al., P.N.A.S (USA) 92:10, 909-13 (1995), "Phage display selection of ligand residues important for Src homology 3 domain binding specificty" Feng. S., et al., P.N.A.S (USA) 92:12,408-15 (1995), "Specific interactions outside the proline-rich core of two classes of Src homology 3 ligands"

## Claims

1. A peptide of 11 to 20 amino acid residues, the peptide being conjugated with mono- or di-palmitic acid to its N-terminal lysine, having the amino acid sequence represented by the following formula (I):
KX₁X₂X₃PX₄X₅PX₆PX₇ (I)
Wherein, K is Lys;
P is Pro,
X1 is Glu, Arg, Asp or Lys;
X2 is Arg, Lys, Pro, Leu or Ile;
X3, X4 and X5 are, identical or different from each other, independently Pro,
Met, Val, Trp, Phe, Ile or Leu;
X6 is Asn, Gln, Art or Lys; and
X7 is Asp, Glu, Arg or Lys;
wherein, said peptide disrupts intracellular signal transduction mediated by proteins including SH3 domains by binding to SH3-domains.

2. The peptide according to claim 1, which is one of SEQ. ID. NO. 1 through SEQ. ID. NO. 12.

3. The peptide according to any one of claim 1 to claim 2, wherein the amino acid residues are L-amino acids.

4. The peptide according to claim 3, wherein one or more of amino acid residues are substituted with D-amino acid.

5. The peptide according to any one of claim 1 to claim 4, wherein one or more of amino acid residues are substituted with hydroxyproline, hydroxylysine, cistine, throxine, norleucine, pyroglutamic acid, or methylated amino acids.

6. The peptide according to any one of claim 1 to claim 5, wherein C-terminal of the peptide is in the form of amide, carboxyl or ester.

7. The peptide according to any one of claim 1 to claim 6, wherein it is coupled to palmitoyl-palmitoyl-lysine, wherein the Lys residue is the Lys present in formula (I).

8. A pharmaceutical composition for preventing or treating cancer or immunosuppression-related diseases, which comprises one or more peptides of any one of claim 1 to claim 7 as an active ingredient and pharmaceutically acceptable carriers.

## Patentansprüche

1. Peptid aus 11 bis 20 Aminosäureresten, wobei das Peptid mit einer Mono- oder Di-Palmitinsäure an seinem N-terminalen Lysin konjugiert ist und die durch die folgende Formel (I) dargestellte Aminosäuresequenz aufweist:
KX₁X₂X₃PX₄X₅PX₆PX₇ (I),
wobei K Lys ist,
P Pro ist,
X1 Glu, Arg, Asp oder Lys ist,
X2 Arg, Lys, Pro, Leu oder Ile ist,
X3, X4 und X5, identisch oder unterschiedlich voneinander, unabhängig Pro,
Met, Val, Trp, Phe, Ile oder Leu sind,
X6 Asn, Gln, Art oder Lys ist und
X7 Asp, Glu, Arg oder Lys ist,
wobei das Peptid die intrazelluläre Signalübertragung, die durch Proteine vermittelt wird, die SH3-Domänen aufweisen, durch Bindung an SH3-Domänen unterbricht.

2. Peptid nach Anspruch 1, das eines der SEQ. ID. NO. 1 bis SEQ. ID. NO. 12 ist.

3. Peptid nach einem der Ansprüche 1 bis 2, wobei die Aminosäurereste L-Aminosäuren sind.

4. Peptid nach Anspruch 3, wobei einer oder mehrere der Aminosäurereste durch eine D-Aminosäure substituiert sind.

5. Peptid nach einem der Ansprüche 1 bis 4, wobei einer oder mehrere der Aminosäurereste durch Hydroxyprolin, Hydroxylysin, Cystin, Throxin, Norleucin, Pyroglutaminsäure oder methylierte Aminosäuren substituiert sind.

6. Peptid nach einem der Ansprüche 1 bis 5, wobei das C-Terminal des Peptids in der Form von Amid, Carboxyl oder Ester vorliegt.

7. Peptid nach einem der Ansprüche 1 bis 6, wobei es an Palmitoyl-palmitoyl-Lysin gekoppelt ist, wobei der Lys-Rest das in der Formel (I) vorliegende Lys ist.

8. Pharmazeutische Zusammensetzung zur Vorbeugung oder Behandlung von Krebs oder von durch eine Immunsupprimierung hervorgerufenen Erkrankungen, die ein oder mehrere Peptide eines der Ansprüche 1 bis 7 als einen aktiven Bestandteil und pharmazeutisch akzeptable Träger aufweist.

## Revendications

1. Un peptide de 11 à 20 résidus acide aminé, le peptide étant conjugué à un acide mono ou dipalmitique à sa fin de chaîne N lysine, ayant la séquence acide aminé représentée par la formule (1) :
**KX**_{**1**}**X**_{**2**}**X**_{**3**}**PX**_{**4**}**X**_{**s**}**PX**_{**6**}**PX**_{**7**} (1)
dans laquelle, K est Lys,
P est Pro,
X1 est Glu, Arg, Asp ou Lys,
X2 est Arg, Lys, Pro, Leu ou Ile,
X3, X4 et X5 sont, identiques ou différents les uns des autres, indépendamment Pro, Met, Val, Trp , Phe , Ile ou Leu,
X6 est Asn, Gln, Art ou Lys et
X7 est Asp, Glu, Arg ou Lys ;
dans lequel ledit peptide rompt une transduction de signal intracellulaire interposé par des protéines incluant des domaines SH3 en liant à des domaines SH3.

2. Le peptide selon la revendication 1,
qui est un de SEQ. ID. NO.1 à SEQ. ID. NO. 12.

3. Le peptide selon l'une quelconque de la revendication 1 et de la revendication 2,
dans lequel les résidus acide aminé sont des acides L-aminés.

4. Le peptide selon la revendication 3,
dans lequel un ou plusieurs des résidus acide aminé sont substitués par un acide D-aminé.

5. Le peptide selon l'une quelconque des revendications 1 à 4,
dans lequel un ou plusieurs résidus acide aminé sont substitués par de l'hydroxyproline, de l'hydroxylysine, de la cistine, de la throxine, de la norceuline, de l'acide pyroglutamique ou des acides aminés méthylés.

6. Le peptide selon l'une des revendications 1 à 5,
dans lequel la fin de chaîne C du peptide est sous la forme amide, carboxyle ou ester.

7. Le peptide selon l'une quelconque des revendications 1 à 6,
dans lequel il est couplé à une palmitoyl-palmitoyl-lysine, dans laquelle le résidu Lys est le Lys présent dans la formule (1).

8. Une composition pharmaceutique pour prévenir ou traiter le cancer ou des maladies reliées à une immunosuppression,
qui comprend un ou plusieurs peptides de l'une quelconque de la revendication 1 à la revendication 7 en tant qu'ingrédient actif et supports pharmaceutiquement acceptables.
